# EUROPEAN PATENT APPLICATION

(11) **EP 1 767 163 A1**
(43) Date of publication of application: **28.03.2007**
(21) Application number: 05020665.5
(22) Date of filing: 22.09.2005
(51) Int. Cl.: A61B 18/14

(54) **Bipolar forceps with multiple electrode array end effector assembly**

(71) Applicant: Sherwood Services AG, 8200 Schaffhausen (CH)
(72) Inventor: Odom, Darren, Longmont, Colorado 80501 (US); Hammill, Curt, Erie, Colorado 80516 (US); Schechter, David A., Longmont, Colorado (US); Tetzlaff, Philip, Superior, Colorado (US); Roy, Jeffrey M., Boulder, Colorado (US)
(74) Representative: HOFFMANN EITLE

(57) **Abstract**

A bipolar electrosurgical forceps includes first and second opposing jaw members having respective inwardly facing surfaces associated therewith. The first and second jaw members are adapted for relative movement between an open position to receive tissue and a closed position engaging tissue between the inwardly facing surfaces. The first and second jaw members each include a plurality of electrodes on the inwardly facing surfaces. The plurality of electrodes of the first jaw member are disposed in substantially vertical registration with the plurality of electrodes of the second jaw member. Each of the plurality of electrodes is configured to connect to a source of electrosurgical energy. Electrodes on at least one jaw member are grouped in pairs and each respective pair aligns with at least one electrode on the opposite jaw member. A multiplexer controls current density or activation sequence of the electrosurgical energy to each electrode.

## Description

### BACKGROUND

The present disclosure relates to forceps used for open and/or endoscopic surgical procedures. More particularly, the present disclosure relates to a forceps which applies a unique combination of mechanical clamping pressure and electrosurgical current to micro-seal soft tissue to promote tissue healing.

### Technical Field

A hemostat or forceps is a simple plier-like tool which uses mechanical action between its jaws to constrict vessels and is commonly used in open surgical procedures to grasp, dissect and/or clamp tissue. Electrosurgical forceps utilize both mechanical clamping action and electrical energy to effect hemostasis by heating the tissue and blood vessels to coagulate, cauterize and/or seal tissue. The electrode of each opposing jaw member is charged to a different electric potential such that when the jaw members grasp tissue, electrical energy can be selectively transferred through the tissue. A surgeon can either cauterize, coagulate/desiccate and/or simply reduce or slow bleeding, by controlling the intensity, frequency and duration of the electrosurgical energy applied between the electrodes and through the tissue.

For the purposes herein, the term "cauterization" is defined as the use of heat to destroy tissue (also called "diathermy" or "electrodiathermy"). The term "coagulation" is defined as a process of desiccating tissue wherein the tissue cells are ruptured and dried. "Vessel sealing" is defined as the process of liquefying the collagen, elastin and ground substances in the tissue so that it reforms into a fused mass with significantly-reduced demarcation between the opposing tissue structures (opposing walls of the lumen). Coagulation of small vessels is usually sufficient to permanently close them. Larger vessels or tissue need to be sealed to assure permanent closure.

Commonly-owned U.S. Application Serial Nos. PCT Application Serial No. PCT/US01/11340 filed on April 6, 2001 by Dycus, et al. entitled "VESSEL SEALER AND DIVIDER", U.S. Application Serial No. 10/116,824 filed on April 5, 2002 by Tetzlaff et al. entitled "VESSEL SEALING INSTRUMENT" and PCT Application Serial No. PCT/US01/11420 filed on April 6, 2001 by Tetzlaff et al. entitled "VESSEL SEALING INSTRUMENT" teach that to effectively seal tissue or vessels, especially large vessels, two predominant mechanical parameters must be accurately controlled: 1) the pressure applied to the vessel; and 2) the gap distance between the conductive tissue contacting surfaces (electrodes). As can be appreciated, both of these parameters are affected by the thickness of the vessel or tissue being sealed. Accurate application of pressure is important for several reasons: to oppose the walls of the vessel; to reduce the tissue impedance to a low enough value that allows enough electrosurgical energy through the tissue; to overcome the forces of expansion during tissue heating; and to contribute to the end tissue thickness which is an indication of a good seal. It has been determined that a typical sealed vessel wall is optimum between 0.001 inches and 0.006 inches. Below this range, the seal may shred or tear and above this range the lumens may not be property or effectively sealed.

With respect to smaller vessels, the pressure applied become less relevant and the gap distance between the electrically conductive surfaces becomes more significant for effective sealing. In other words, the chances of the two electrically conductive surfaces touching during activation increases as the tissue thickness and the vessels become smaller.

As can be appreciated, when cauterizing, coagulating or sealing vessels, the tissue disposed between the two opposing jaw members is essentially destroyed (e.g., heated, ruptured and/or dried with cauterization and coagulation and fused into a single mass with vessel sealing). Other known electrosurgical instruments include blade members or shearing members which simply cut tissue in a mechanical and/or electromechanical manner and, as such, also destroy tissue viability.

When trying to electrosurgically treat large, soft tissues (e.g., lung, intestine, lymph ducts, etc.) to promote healing, the above-identified surgical treatments are generally impractical due to the fact that in each instance the tissue or a significant portion thereof is essentially destroyed to create the desired surgical effect, cauterization, coagulation and/or sealing. As a result thereof, the tissue is no longer viable across the treatment site, i.e., there remains no feasible path across the tissue for vascularization.

Thus, a need exists to develop an electrosurgical forceps which effectively treats tissue while maintaining tissue viability across the treatment area to promote tissue healing.

A need exists also to enhance sealing strength in tissue fusion by increasing resistance to fluid flow or increased pressure at the fusion site so as to minimize entry of fluid into the perimeter of the fused site during burst strength testing. The entry of fluid often results in seal failure due to propagation of the fluid to the center of the tissue seal.

In addition, a need exists lengthen the jaws of existing electrosurgical forceps beyond current mechanical limits so as to increase current density to reduce sealing time, and increase tissue desiccation and seal strength.

### SUMMARY

It is an object of the present disclosure to provide a bipolar electrosurgical forceps having jaw members which are configured with electrode surfaces with a plurality of flow paths so as to increase resistance to fluid flow through the tissue seal zone, or increasing pressure states at the fusion site, thereby increasing tissue seal integrity.

It is an object of the present disclosure to reduce mechanical tolerance requirements of a bipolar electrosurgical forceps while maintaining or increasing current density by providing jaw members which are longer than those of the prior art.

It is an object of the present disclosure to provide a bipolar electrosurgical forceps having a plurality of electrodes on each jaw member to form an array of individual pairs of corresponding or counterpart electrodes on each jaw member so that the activation sequence and electrosurgical energy applied to each individual pair of corresponding or counterpart electrodes may be varied to maintain or increase pressure of the tissue during tissue desiccation, thereby increasing tissue seal integrity.

The present disclosure relates to a bipolar electrosurgical forceps, which includes first and second opposing jaw members having respective inwardly facing surfaces associated therewith. The first and second jaw members are adapted for relative movement between an open position to receive tissue and a closed position engaging tissue between the inwardly facing surfaces. The first and second jaw members each include a plurality of electrodes on the inwardly facing surfaces thereof. The plurality of electrodes of the first jaw member are disposed in substantially vertical registration with the plurality of electrodes of the second jaw member, and each of the plurality of electrodes is configured to connect to a source of electrosurgical energy.

In one embodiment, electrodes on at least one jaw member may be grouped in pairs and each respective pair may be aligned with at least one electrode on the opposite jaw member. Each pair of electrodes on each jaw member may be disposed in substantially vertical registration with a corresponding pair of electrodes on the opposite jaw member. A series of leads may couple each electrode to an electrosurgical generator via at least one multiplexer coupled therebetween. The series of leads may be coupled to the multiplexer and the multiplexer controls electrosurgical energy to each electrode. The multiplexer may control at least one of current density and activation sequence of the electrosurgical energy to each electrode. The plurality of electrodes may be configured in a staggered arrangement with respect to one another on each jaw member.

The present disclosure relates also to a method of sealing tissue with a bipolar electrosurgical forceps. The method includes the steps of: providing a forceps having an end effector assembly with first and second jaw members including opposing inwardly-facing surfaces each including a plurality of electrodes disposed thereon. The plurality of electrodes on the inwardly facing surface of the first jaw member are in substantially vertical registration with the plurality of electrodes on the inwardly facing surface of the second jaw member to form an opposing electrode pair. Each electrode is individually configured to a source of electrosurgical energy. Additionally, the method includes the steps of grasping tissue between the jaw member and selectively applying electrosurgical energy to the electrodes according to an algorithm which controls the activation of each electrode.

In one embodiment, the method may further include the steps of: decreasing electrosurgical energy to at least one of the opposing electrode pairs; and increasing electrosurgical energy to at least one other opposing electrode pair. In addition, the method may further include the steps of applying electrosurgical energy by advancing in progression along respective opposing electrode pairs from a distal end of the end effector assembly to a proximal end of the end effector assembly.

### BRIEF DESCRIPTION OF THE DRAWINGS

Various embodiments of the subject instrument are described herein with reference to the drawings wherein:
FIG. 1A is a perspective view of an endoscopic forceps having an electrode assembly in accordance with one embodiment of the present disclosure;
FIG. 1B is a perspective view of an open forceps having a electrode assembly in accordance with one embodiment of the present disclosure;
FIG. 2 is an enlarged, perspective view of the electrode assembly of the forceps of FIG. 1B shown in an open configuration;
FIG. 3A is an enlarged, schematic view of one embodiment of the electrode assembly showing a pair of opposing, concentrically-oriented electrodes disposed on a pair of opposing jaw members;
FIG. 3B is a partial, side cross-sectional view of the electrode assembly of FIG. 3A;
FIG. 4A is an enlarged, schematic view of another embodiment of the electrode assembly showing a plurality of concentrically-oriented electrode micro-sealing pads disposed on the same jaw member;
FIG. 4B is a greatly enlarged view of the area of detail in FIG. 4A showing the electrical path during activation of the electrode assembly;
FIG. 4C is an enlarged schematic view showing the individual micro-sealing sites and viable tissue areas between the two jaw members after activation;
FIG. 5A is a schematic, perspective view of the jaw members approximating tissue;
FIG. 5B is a schematic, perspective view of the jaw members grasping tissue; and
FIG. 5C is a schematic, perspective view showing a series of micro-seals disposed in a pattern across the tissue after activation of the electrode assembly.
FIG. 6 is plan view of a tissue seal sealed by an electrosurgical forceps according to the prior art showing a potentially weaker seal area due to fluid entry into the seal perimeter;
FIG. 7A is a partially schematic top plan view of a jaw member of an electrosurgical forceps according to another embodiment of the present disclosure and showing the electrical power supply to the jaw member;
FIG. 7B is a partially schematic bottom plan view of a jaw member of an electrosurgical forceps according to another embodiment of the present disclosure and showing the electrical power supply to the jaw member;
FIG. 8 is an elevation view of jaw members of an electrosurgical forceps of FIGS. 7A and 7B grasping tissue; and
FIG. 9 is a plan view of a tissue seal sealed by an electrosurgical forceps according to the present disclosure of FIG. 8.

### DETAILED DESCRIPTION

This application incorporates by reference herein in its entirety commonly owned, concurrently filed, co-pending U.S. Patent Application Serial No. (attorney docket no.: 2886 PCT CIP II (203-3427 PCT CIP II)) by Hammill et al entitled "ELECTRODE ASSEMBLY FOR TISSUE FUSION."

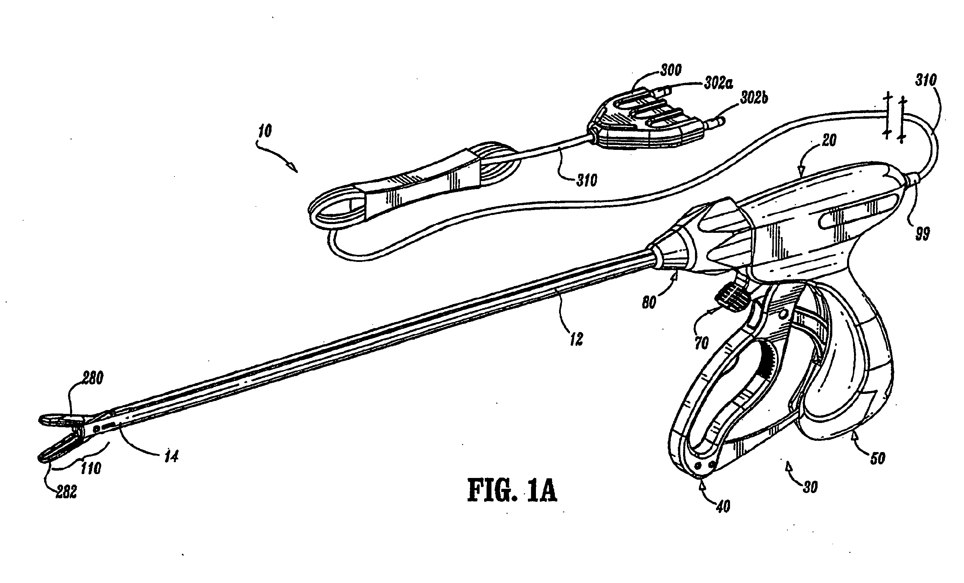

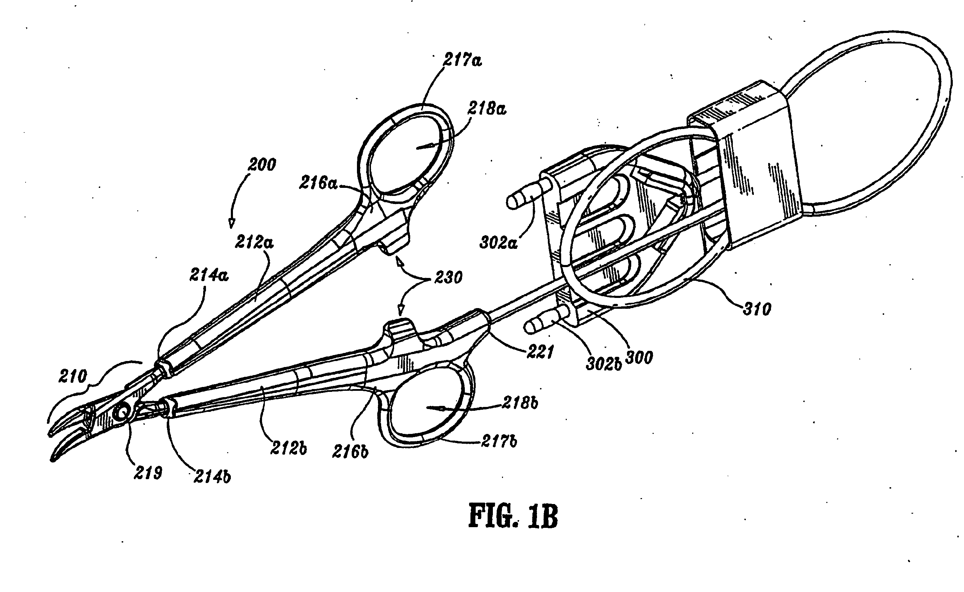

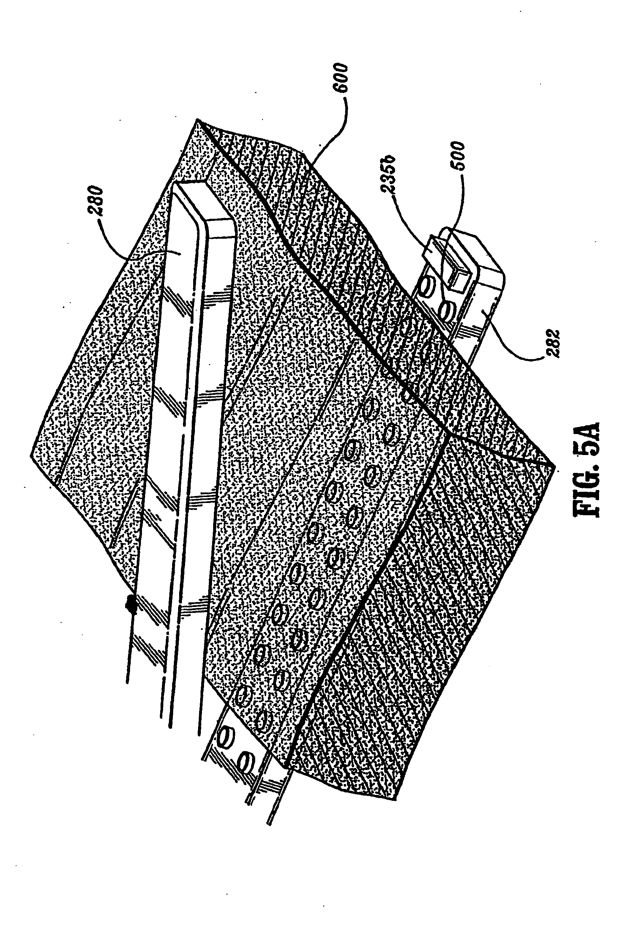

Referring now to FIG. 1A, a bipolar forceps 10 is shown for use with various surgical procedures. Forceps 10 generally includes a housing 20, a handle assembly 30, a rotating assembly 80, an activation assembly 70 and an electrode assembly 110 which mutually cooperate to grasp and seal tissue 600 (See FIGS. 5A-5C). Although the majority of the figure drawings depict a bipolar forceps 10 for use in connection with endoscopic surgical procedures, an open forceps 200 is also contemplated for use in connection with traditional open surgical procedures and is shown by way of example in FIG. 1B and is described below. For the purposes herein, either an endoscopic instrument or an open instrument may be utilized with the electrode assembly described herein. Obviously, different electrical and mechanical connections and considerations apply to each particular type of instrument, however, the novel aspects with respect to the electrode assembly and its operating characteristics remain generally consistent with respect to both the open or endoscopic designs.

More particularly, forceps 10 includes a shaft 12 which has a distal end 14 dimensioned to mechanically engage a jaw assembly 110 and a proximal end 16 which mechanically engages the housing 20. The shaft 12 may be bifurcated at the distal end 14 thereof to receive the jaw assembly 110. The proximal end 16 of shaft 12 mechanically engages the rotating assembly 80 to facilitate rotation of the jaw assembly 110. In the drawings and in the descriptions which follow, the term "proximal", as is traditional, will refer to the end of the forceps 10 which is closer to the user, while the term "distal" will refer to the end which is further from the user.

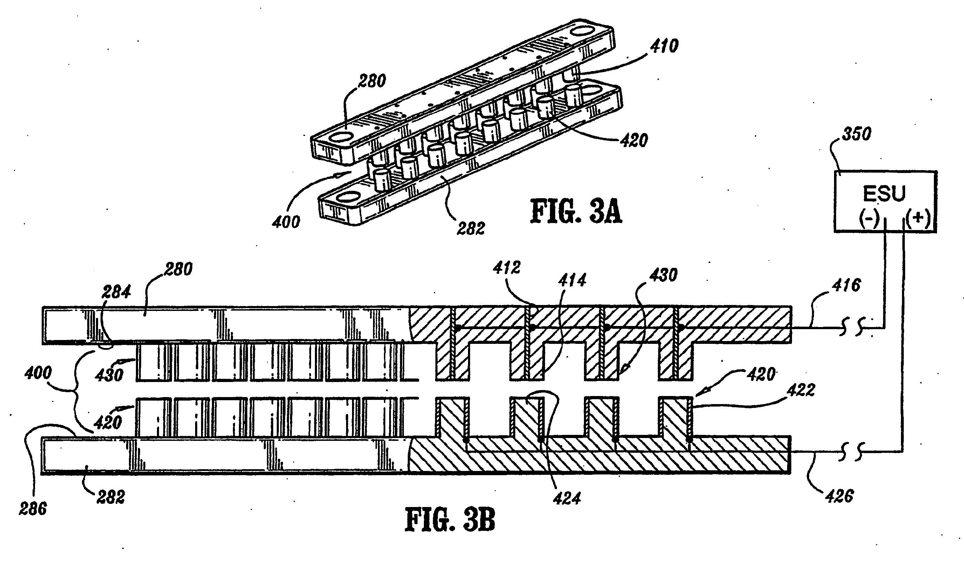

Forceps 10 also includes an electrical interface or plug 300 which connects the forceps 10 to a source of electrosurgical energy, e.g., an electrosurgical generator 350 (See FIG. 3B). Plug 300 includes a pair of prong members 302a and 302b which are dimensioned to mechanically and electrically connect the forceps 10 to the electrosurgical generator 350. An electrical cable 310 extends from the plug 300 to a sleeve 99 which securely connects the cable 310 to the forceps 10. Cable 310 is internally divided within the housing 20 to transmit electrosurgical energy through various electrical feed paths to the jaw assembly 110 as explained in more detail below.

Handle assembly 30 includes a fixed handle 50 and a movable handle 40. Fixed handle 50 is integrally associated with housing 20 and handle 40 is movable relative to fixed handle 50 to actuate a pair of opposing jaw members 280 and 282 of the jaw assembly 110 as explained in more detail below. The activation assembly 70 is selectively movable by the surgeon to energize the jaw assembly 110. Movable handle 40 and activation assembly 70 are preferably of unitary construction and are operatively connected to the housing 20 and the fixed handle 50 during the assembly process.

As mentioned above, jaw assembly 110 is attached to the distal end 14 of shaft 12 and includes a pair of opposing jaw members 280 and 282. Movable handle 40 of handle assembly 30 imparts movement of the jaw members 280 and 282 about a pivot pin 119 from an open position wherein the jaw members 280 and 282 are disposed in spaced relation relative to one another for approximating tissue 600, to a clamping or closed position wherein the jaw members 280 and 282 cooperate to grasp tissue 600 therebetween (See FIGS. 5A-5C).

It is envisioned that the forceps 10 may be designed such that it is fully or partially disposable depending upon a particular purpose or to achieve a particular result. For example, jaw assembly 110 may be selectively and releasably engageable with the distal end 14 of the shaft 12 and/or the proximal end 16 of shaft 12 may be selectively and releasably engageable with the housing 20 and the handle assembly 30. In either of these two instances, the forceps 10 would be considered "partially disposable" or "reposable", i.e., a new or different jaw assembly 110 (or jaw assembly 110 and shaft 12) selectively replaces the old jaw assembly 110 as needed.

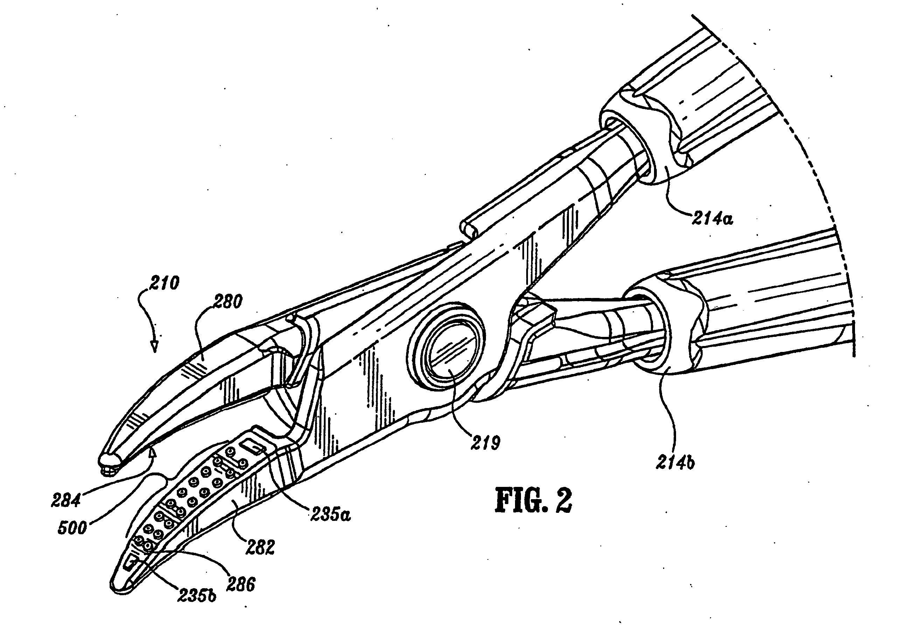

Referring now to FIGS. 1B and 2, an open forceps 200 includes a pair of elongated shaft portions 212a each having a proximal end 216a and 216b, respectively, and a distal end 214a and 214b, respectively. The forceps 200 includes jaw assembly 210 which attaches to distal ends 214a and 214b of shafts 212a and 212b, respectively. Jaw assembly 210 includes opposing jaw members 280 and 282 which are pivotably connected about a pivot pin 219.

Each shaft 212a and 212b includes a handle 217a and 217b disposed at the proximal end 216a and 216b thereof which each define a finger hole 218a and 218b, respectively, therethrough for receiving a finger of the user. As can be appreciated, finger holes 218a and 218b facilitate movement of the shafts 212a and 212b relative to one another which, in turn, pivot the jaw members 280 and 282 from an open position wherein the jaw members 280 and 282 are disposed in spaced relation relative to one another for approximating tissue 600 to a clamping or closed position wherein the jaw members 280 and 282 cooperate to grasp tissue 600 therebetween. A ratchet 230 is typically included for selectively locking the jaw members 280 and 282 relative to one another at various positions during pivoting.

Typically, each position associated with the cooperating ratchet interfaces 230 holds a specific, i.e., constant, strain energy in the shaft members 212a and 212b which, in turn, transmits a specific closing force to the jaw members 280 and 282. It is envisioned that the ratchet 230 may include graduations or other visual markings which enable the user to easily and quickly ascertain and control the amount of closure force desired between the jaw members 280 and 282.

One of the shafts, e.g., 212b, includes a proximal shaft connector /flange 221 which is designed to connect the forceps 200 to a source of electrosurgical energy such as an electrosurgical generator 350 (FIG. 3B). More particularly, flange 221 mechanically secures electrosurgical cable 310 to the forceps 200 such that the user may selectively apply electrosurgical energy as needed. The proximal end of the cable 310 includes a similar plug_300 as described above with respect to FIG. 1A. The interior of cable 310 houses a pair of leads which conduct different electrical potentials from the electrosurgical generator 350 to the jaw members 280 and 282 as explained below with respect to FIG. 2.

The jaw members 280 and 282 are generally symmetrical and include similar component features which cooperate to permit facile rotation about pivot 219 to effect the grasping of tissue 600. Each jaw member 280 and 282 includes a non-conductive tissue contacting surface 284 and 286, respectively, which cooperate to engage the tissue 600 during treatment.

As best shown in FIG. 2, the various electrical connections of the electrode assembly 210 are preferably configured to provide electrical continuity to an array of electrode micro-sealing pads 500 of disposed across one or both jaw members 280 and 282. The electrical paths 416, 426 or 516, 526 from the array of electrode micro-sealing pads 500 are preferably mechanically and electrically interfaced with corresponding electrical connections (not shown) disposed within shafts 212a and 212b, respectively. As can be appreciated, these electrical paths 416, 426 or 516, 526 may be permanently soldered to the shafts 212a and 212b during the assembly process of a disposable instrument or, alternatively, selectively removable for use with a reposable instrument.

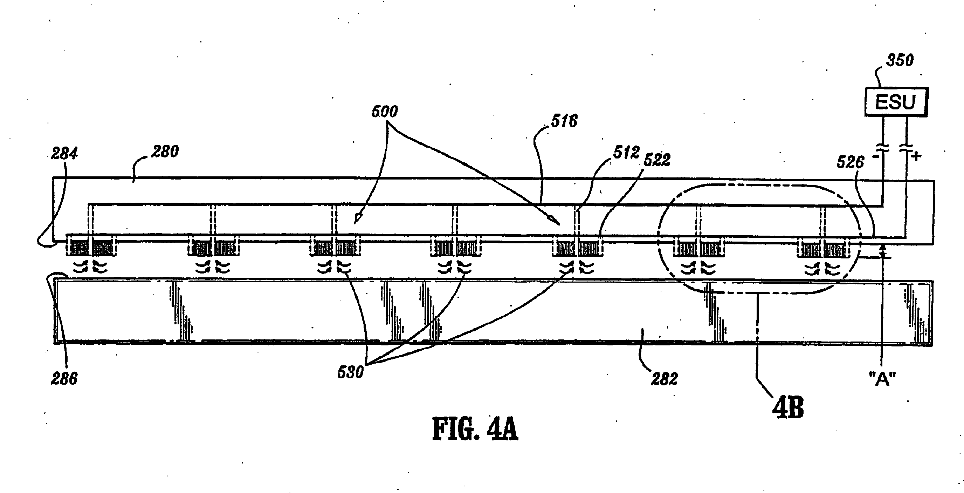

As best shown in FIGS. 4A-4C, the electrical paths are connected to the plurality of electrode micro-sealing pads 500 within the jaw assembly 210. More particularly, the first electrical path 526 (i.e., an electrical path having a first electrical potential) is connected to each ring electrode 522 of each electrode micro-sealing pad 500. The second electrical path 516 (i.e., an electrical path having a second electrical potential) is connected to each post electrode 522 of each electrode micro-sealing pad 500.

The electrical paths 516 and 526 typically do not encumber the movement of the jaw members 280 and 282 relative to one another during the manipulation and grasping of tissue 400. Likewise, the movement of the jaw members 280 and 282 do not unnecessarily strain the electrical paths 516 and 526 or their respective connections 517, 527.

As best seen in FIGS. 2-5C, jaw members 280 and 282 both include non-conductive tissue contacting surfaces 284 and 286, respectively, disposed along substantially the entire longitudinal length thereof (i.e., extending substantially from the proximal to distal end of each respective jaw member 280 and 284). The non-conductive tissue contacting surfaces 284 and 286 may be made from an insulative material such as ceramic due to its hardness and inherent ability to withstand high temperature fluctuations. Alternatively, the non-conductive tissue contacting surfaces 284 and 286 may be made from a material or a combination of materials having a high Comparative Tracking Index (CTI) in the range of about 300 to about 600 volts. Examples of high CTI materials include nylons and syndiotactic polystryrenes such as QUESTRA^{®} manufactured by DOW Chemical. Other materials may also be utilized either alone or in combination, e.g., Nylons, Syndiotactic-polystryrene (SPS), Polybutylene Terephthalate (PBT), Polycarbonate (PC), Acrylonitrile Butadiene Styrene (ABS), Polyphthalamide (PPA), Polymide, Polyethylene Terephthalate (PET), Polyamide-imide (PAI), Acrylic (PMMA), Polystyrene (PS and HIPS), Polyether Sulfone (PES), Aliphatic Polyketone, Acetal (POM) Copolymer, Polyurethane (PU and TPU), Nylon with Polyphenylene-oxide dispersion and Acrylonitrile Styrene Acrylate. Preferably, the non-conductive tissue contacting surfaces 284 and 286 are dimensioned to securingly engage and grasp the tissue 600 and may include serrations (not shown) or roughened surfaces to facilitate approximating and grasping tissue.

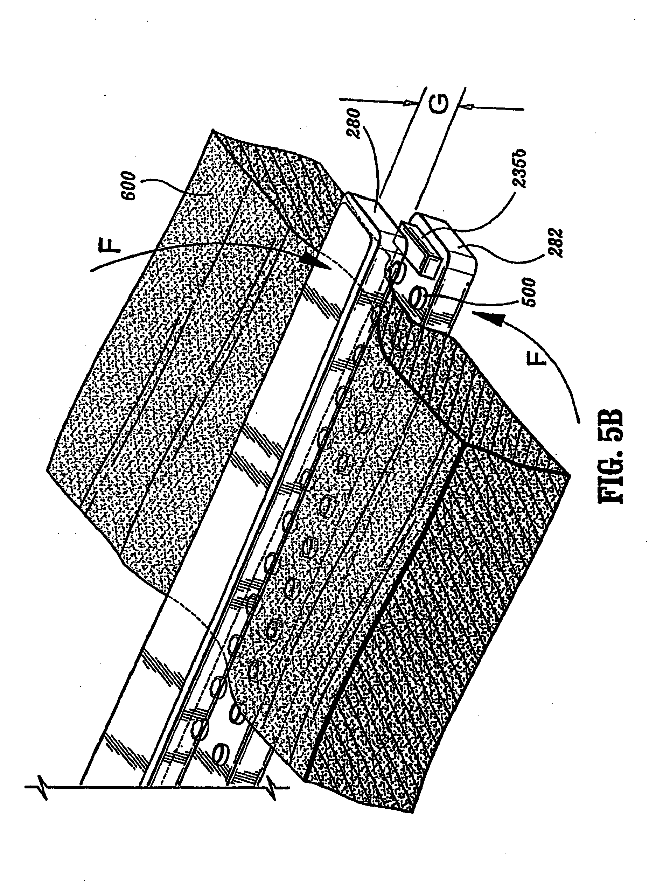

It is envisioned that one of the jaw members, e.g., 282, includes at least one stop member 235a, 235b (FIG. 2) disposed on the inner facing surface of the sealing surfaces 286. Alternatively or in addition, one or more stop members 235a, 235b may be positioned adjacent to the non-conductive sealing surfaces 284, 286 or proximate the pivot 219. The stop members 235a, 235b are preferably designed to define a gap "G" (FIG. 5B) between opposing jaw members 280 and 282 during the micro-sealing process. The separation distance during micro-sealing or the gap distance "G" is within the range of about 0.001 inches (∼0.03 millimeters) to about 0.006 inches (∼0.016 millimeters). One or more stop members 235a, 235b may be positioned on the distal end and proximal end of one or both of the jaw members 280, 282 or may be positioned between adjacent electrode micro-sealing pads 500. Moreover, the stop members 235a and 235b may be integrally associated with the non-conductive tissue contacting surfaces 284 and 286. It is envisioned that the array of electrode micro-sealing pads 500 may also act as stop members for regulating the distance "G" between opposing jaw members 280, 282 (See FIG. 4C).

As mentioned above, the effectiveness of the resulting micro-seal is dependent upon the pressure applied between opposing jaw members 280 and 282, the pressure applied by each electrode micro-sealing pad 500 at each micro-sealing site 620 (FIG. 4C), the gap "G" between the opposing jaw members 280 and 282 (either regaled by a stop member 235a, 235b or the array of electrode micro-sealing pads 500) and the control of the electrosurgical intensity during the micro-sealing process. Applying the correct force is important to oppose the walls of the tissue; to reduce the tissue impedance to a low enough value that allows enough current through the tissue; and to overcome the forces of expansion during tissue heating in addition to contributing towards creating the required end tissue thickness which is an indication of a good micro-seal. Regulating the gap distance and regulating the electrosurgical intensity ensure a consistent seal quality and reduce the likelihood of collateral damage to surrounding tissue.

As best shown in FIG. 2, the electrode micro-sealing pads 500 are arranged in a longitudinal, pair-like fashion along the tissue contacting surfaces 286 and/or 284. Two or more micro-sealing pads 500 may extend transversally across the tissue contacting surface 286. FIGS. 3A and 3B show one embodiment of the present disclosure wherein the electrode micro-sealing pads 500 include a ring electrode 422 disposed on one jaw members 282 and a post electrode 412 disposed on the other jaw member 280. The ring electrode 422 includes an insulating material 424 disposed therein to form a ring electrode and insulator assembly 420 and the post electrode 422 includes an insulating material disposed therearound to form a post electrode and insulator assembly 430. Each post electrode assembly 430 and the ring electrode assembly 420 of this embodiment together define one electrode micro-sealing pad 400. Although shown as a circular-shape, ring electrode 422 may assume any other annular or enclosed configuration or alternatively partially enclosed configuration such as a C-shape arrangement.

As best shown in FIG. 3B, the post electrode 422 is concentrically centered opposite the ring electrode 422 such that when the jaw members 280 and 282 are closed about the tissue 600, electrosurgical energy flows from the ring electrode 422, through tissue 600 and to the post electrode 412. The insulating materials 414 and 424 isolate the electrodes 412 and 422 and prevent stray current tracking to surrounding tissue. Alternatively, the electrosurgical energy may flow from the post electrode 412 to the ring electrode 422 depending upon a particular purpose.

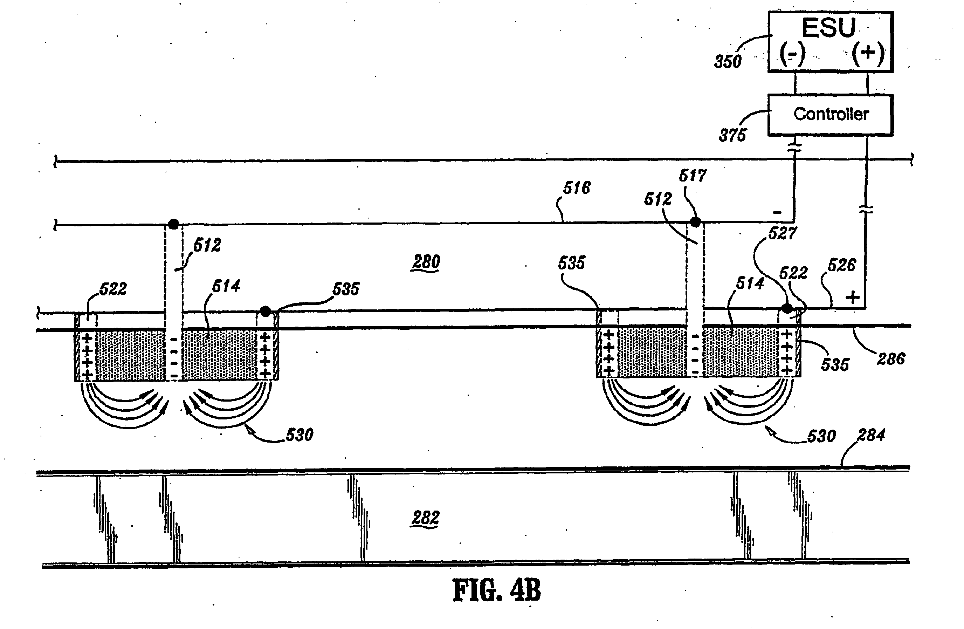

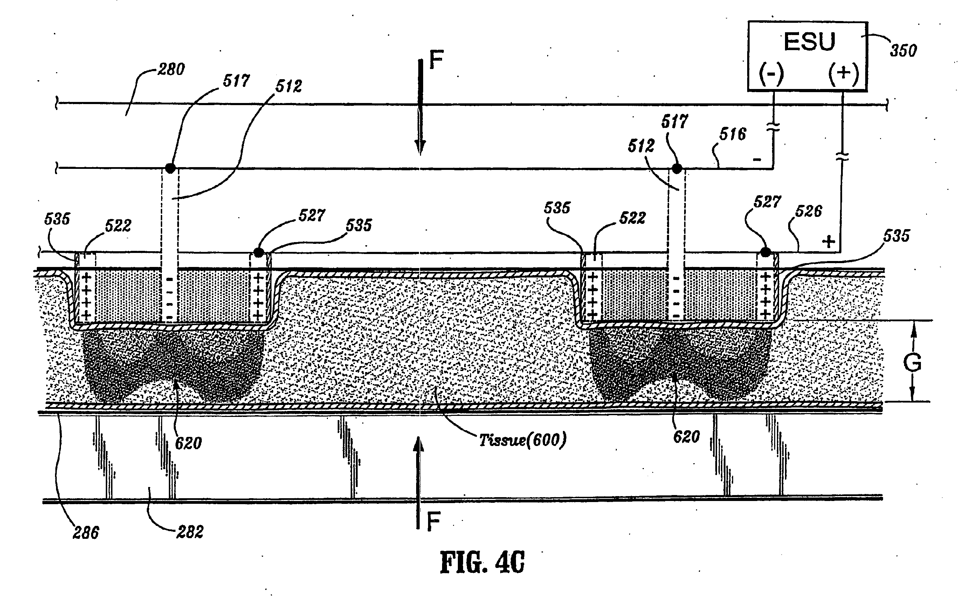

FIGS. 4A-4C show an alternate embodiment of the jaw assembly 210 according to the present disclosure for micro-sealing tissue 600 wherein each electrode micro-sealing pad 500 is disposed on a single jaw member, e.g., jaw member 280. More particularly and as best illustrated in FIG. 4B, each electrode micro-sealing pad 500 consists of an inner post electrode 512 which is surrounded by an insulative material 514, e.g., ceramic. The insulative material 514 is, in turn, encapsulated by a ring electrode 522. A second insulative material 535 (or the same insulative material 514) may be configured to encase the ring electrode 522 to prevent stray electrical currents to surrounding tissue.

The ring electrode 522 is connected to the electrosurgical generator 350 by way of a cable 526 (or other conductive path) which transmits a first electrical potential to each ring electrode 522 at connection 527. The post electrode 512 is connected to the electrosurgical generator 350 by way of a cable 516 (or other conductive path) which transmits a second electrical potential to each post electrode 522 at connection 517. A controller 375 (See FIG. 4B) may be electrically interposed between the generator 350 and the electrodes 512, 522 to regulate the electrosurgical energy supplied thereto depending upon certain electrical parameters, current impedance, temperature, voltage, etc. For example, the instrument or the controller may include one or more smart sensors (not shown) which communicate with the electrosurgical generator 350 (or smart circuit, computer, feedback loop, etc.) to automatically regulate the electrosurgical intensity (waveform, current, voltage, etc.) to enhance the micro-sealing process. The sensor may measure or monitor one or more of the following parameters: tissue temperature, tissue impedance at the micro-seal, change in impedance of the tissue over time and/or changes in the power or current applied to the tissue over time. An audible or visual feedback monitor (not shown) may be employed to convey information to the surgeon regarding the overall micro-seal quality or the completion of an effective tissue micro-seal.

Moreover, a PCB circuit of flex circuit (not shown) may be utilized to provide information relating to the gap distance (e.g., a proximity detector may be employed) between the two jaw members 280 and 282, the micro-sealing pressure between the jaw members 280 and 282 prior to and during activation, load (e.g., strain gauge may be employed), the tissue thickness prior to or during activation, the impedance across the tissue during activation, the temperature during activation, the rate of tissue expansion during activation and micro-sealing. It is envisioned that the PCB circuit may be designed to provide electrical feedback to the generator 350 relating to one or more of the above parameters either on a continuous basis or upon inquiry from the generator 350. For example, a PCB circuit may be employed to control the power, current and/or type of current waveform from the generator 350 to the jaw members 280, 282 to reduce collateral damage to surrounding tissue during activation, e.g., thermal spread, tissue vaporization and/or steam from the treatment site. Examples of a various control circuits, generators and algorithms which may be utilized are disclosed in U.S. Patent No 6,228,080 and U.S. Application Serial No. 10/073,761 the entire contents of both of which are hereby incorporated by reference herein.

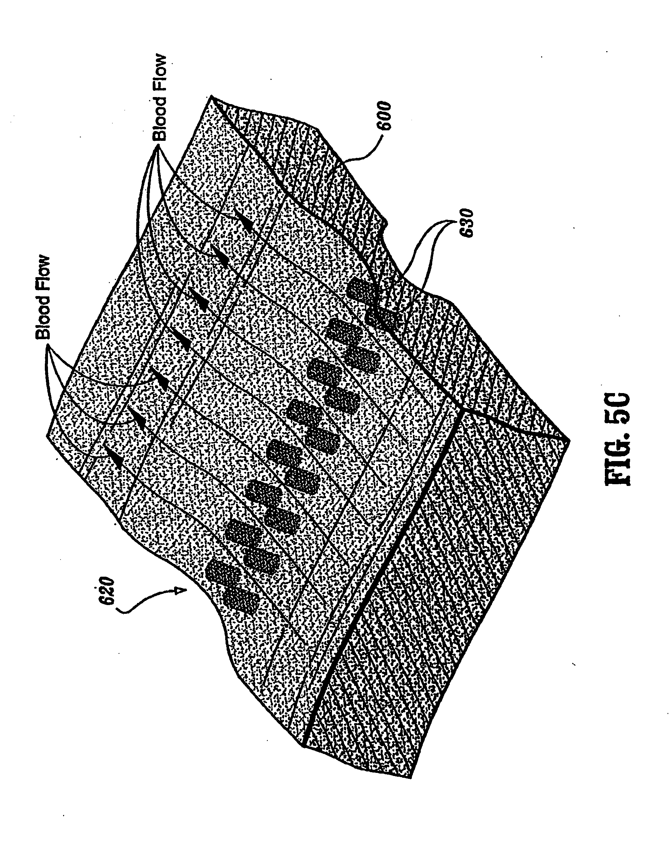

In use as depicted in FIGS. 5A-5C, the surgeon initially approximates the tissue (FIG. 5A) between the opposing jaw member 280 and 282 and then grasps the tissue 600 (FIG. 5B) by actuating the jaw members 280, 282 to rotate about pivot 219. Once the tissue is grasped, the surgeon selectively activates the generator 350 to supply electrosurgical energy to the array of the electrode micro-sealing pads 500. More particularly, electrosurgical energy flows from the ring electrode 522, through the tissue 600 and to the post electrode 512 (See FIGS. 4B and 4C). As a result thereof, an intermittent pattern of individual micro-seals 630 is created along and across the tissue 600 (See FIG. 5C). The arrangement of the micro-sealing pads 500 across the tissue only seals the tissue which is between each micro-sealing pad 500 and the opposing jaw member 282. The adjacent tissue remains viable which, as can be appreciated, allows blood and nutrients to flow through the sealing site 620 and between the individual micro-seals 630 to promote tissue healing and reduce the chances of tissue necrosis. By selectively regulating the closure pressure "F", gap distance "G", and electrosurgical intensity, effective and consistent micro-seals 630 may be created for many different tissue types.

It is further envisioned that selective ring electrodes and post electrodes may have varying electric potentials upon activation. For example, at or proximate the distal tip of one of the jaw members, one or a series of electrodes may be electrically connected to a first potential and the corresponding electrodes (either on the same jaw or perhaps the opposing jaw) may be connected to a second potential. Towards the proximal end of the jaw member, one or a series of electrodes may be connected to a third potential and the corresponding electrodes connected to yet a fourth potential. As can be appreciated, this would allow different types of tissue sealing to take place at different portions of the jaw members upon activation. For example, the type of sealing could be based upon the type of tissues involved or perhaps the thickness of the tissue. To seal larger tissue, the user would grasp the tissue more towards the proximal portion of the opposing jaw members and to seal smaller tissue, the user would grasp the tissue more towards the distal portion of the jaw members. It is also envisioned that the pattern and/or density of the micro-sealing pads may be configured to seal different types of tissue or thicknesses of tissue along the same jaw members depending upon where the tissue is grasped between opposing jaw members.

From the foregoing and with reference to the various figure drawings, those skilled in the art will appreciate that certain modifications can also be made to the present disclosure without departing from the scope of the same. For example, it is envisioned that by making the forceps 100, 200 disposable, the forceps 100, 200 is less likely to become damaged since it is only intended for a single use and, therefore, does not require cleaning or sterilization. As a result, the functionality and consistency of the vital micro-sealing components, e.g., the conductive micro-sealing electrode pads 500, the stop member(s) 235a, 235b, and the insulative materials 514, 535 will assure a uniform and quality seal.

Experimental results suggest that the magnitude of pressure exerted on the tissue by the micro-sealing pads 112 and 122 is important in assuring a proper surgical outcome, maintaining tissue viability. Tissue pressures within a working range of about 3 kg/cm² to about 16 kg/cm² and, preferably, within a working range of 7 kg/cm² to 13 kg/cm² have been shown to be effective for micro-sealing various tissue types and vascular bundles.

In one embodiment, the shafts 212a and 212b are manufactured such that the spring constant of the shafts 212a and 212b, in conjunction with the placement of the interfacing surfaces of the ratchet 230, will yield pressures within the above working range. In addition, the successive positions of the ratchet interfaces increase the pressure between opposing micro-sealing surfaces incrementally within the above working range.

It is envisioned that the outer surface of the jaw members 280 and 282 may include a nickel-based material or coating which is designed to reduce adhesion between the jaw members 280, 282 (or components thereof) with the surrounding tissue during activation and micro-sealing. Moreover, it is also contemplated that other components such as the shaft portions 212a, 212b and the rings 217a, 217b may also be coated with the same or a different "non-stick" material. Preferably, the non-stick materials are of a class of materials that provide a smooth surface to prevent mechanical tooth adhesions.

It is also contemplated that the tissue contacting portions of the electrodes and other portions of the micro-sealing pads 400, 500 may also be made from or coated with non-stick materials. When utilized on these tissue contacting surfaces, the non-stick materials provide an optimal surface energy for eliminating sticking due in part to surface texture and susceptibility to surface breakdown due electrical effects and corrosion in the presence of biologic tissues, It is envisioned that these materials exhibit superior non-stick qualities over stainless steel and should be utilized in areas where the exposure to pressure and electrosurgical energy can create localized "hot spots" more susceptible to tissue adhesion. As can be appreciated, reducing the amount that the tissue "sticks" during micro-sealing improves the overall efficacy of the instrument.

The non-stick materials may be manufactured from one (or a combination of one or more) of the following "non-stick" materials: nickelchrome, chromium nitride, MedCoat 2000 manufactured by The Electrolizing Corporation of OHIO, Inconel 600 and tin-nickel. Inconel 600 coating is a so-called "super alloy" which is manufactured by Special Metals, Inc. located in Conroe Texas. The alloy is primarily used in environments which require resistance to corrosion and heat. The high Nickel content of Inconel 600 makes the material especially resistant to organic corrosion. As can be appreciated, these properties are desirable for bipolar electrosurgical instruments which are naturally exposed to high temperatures, high RF energy and organic matter. Moreover, the resistivity of Inconel 600 is typically higher than the base electrode material which further enhances desiccation and micro-seal quality.

One particular class of materials disclosed herein has demonstrated superior non-stick properties and, in some instances, superior micro-seal quality. For example, nitride coatings which include, but not are not limited to: TiN, ZrN, TiAIN, and CrN are preferred materials used for non-stick purposes. CrN has been found to be particularly useful for non-stick purposes due to its overall surface properties and optimal performance. Other classes of materials have also been found to reducing overall sticking. For example, high nickel/chrome alloys with a Ni/Cr ratio of approximately 5:1 have been found to significantly reduce sticking in bipolar instrumentation.

It is also envisioned that the micro-sealing pads 400, 500 may be arranged in many different configurations across or along the jaw members 280, 282 depending upon a particular purpose. Moreover, it is also contemplated that a knife or cutting element (not shown) may be employed to sever the tissue 600 between a series of micro-sealing pads 400, 500 depending upon a particular purpose. The cutting element may include a cutting edge to simply mechanically cut tissue 600 and/or may be configured to electrosurgically cut tissue 600.

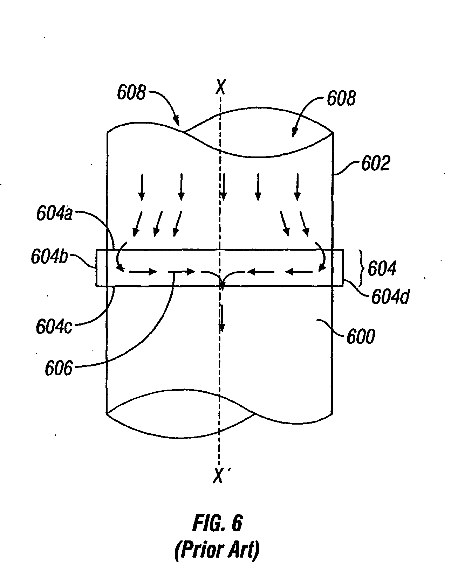

FIG. 6 discloses a resulting tissue seal sealed by an electrosurgical forceps according to the prior art showing a potentially weaker seal area due to fluid entry into the seal perimeter. More particularly, tissue 600 of a lumen 602 of a patient's body such as the large or small intestines or any other passage or vessel is subject to a tissue seal 604 performed by an electrosurgical forceps of the prior art (not shown). The tissue seal 604 may be performed by a heating method. The heating method may include, but is not limited to, radiofrequency (RF), ultrasonic, capacitive or thermoelectric heating methods. The lumen 602 has an approximate centerline axis X-X'. The seal 604 has a perimeter generally of four contiguous sides 604a, 604b, 604c and 604d and a central portion 606. Two sides 604a and 604c extend in a direction generally orthogonal to the centerline axis X-X' of the lumen 602 and parallel to each other, while the two sides 604b and 604d extend in a direction generally parallel to the centerline axis X-X'. It has been determined that during sealing, fluid 608 may enter at a side of the perimeter such as side 604a and propagate to the central portion 606 of the tissue seal 604. A weaker seal may develop as a result of increased fluid in a particular tissue area.

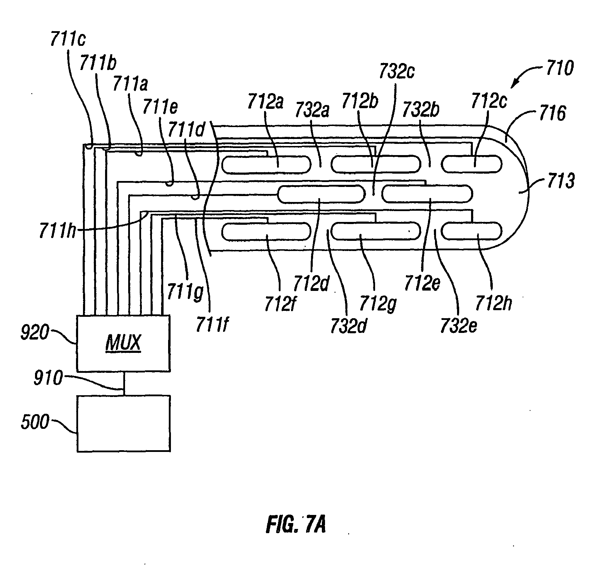

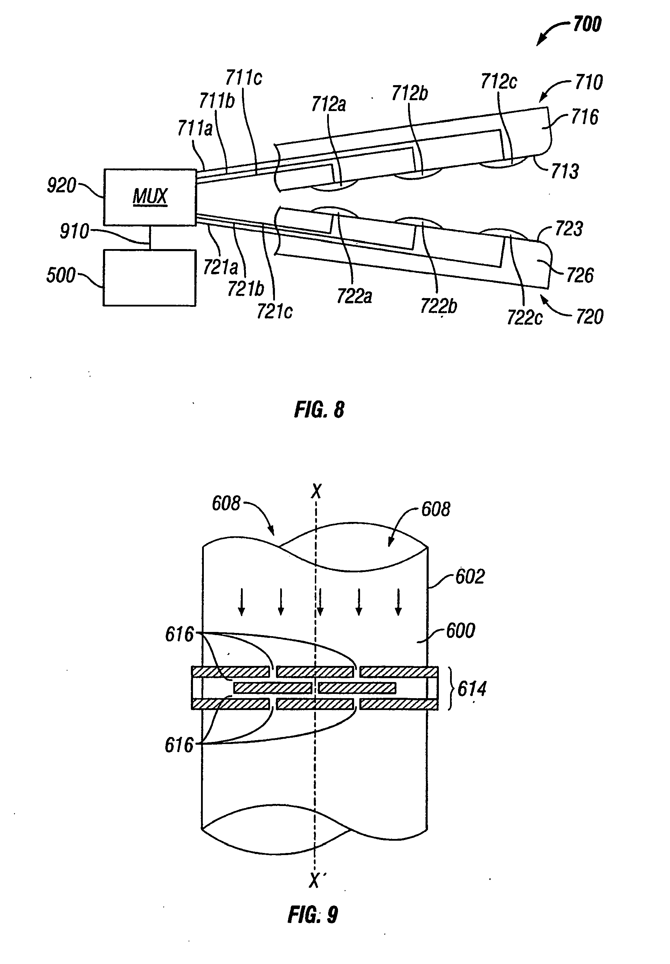

FIGS. 7A-11 illustrate various embodiments of the present disclosure which include an end effector assembly 700 for use with forceps 10. End effector assembly 700 includes jaw members 710 and 720 which cooperate to treat tissue. More particularly and as best shown in FIG. 8, jaw member 710 includes an outer jaw housing 716 which is designed to support a plurality of electrodes 712a, 712b, 712c on an inner facing surface 713 thereof. Likewise, jaw member 720 includes an outer jaw housing 726 which is configured to support a corresponding plurality of electrodes 722a, 722b and 722c on an inner facing surface 723 thereof. The electrodes 712a-712c are typically disposed substantially in general vertical registration relative to one another, however, it is envisioned that the opposing electrodes 722a, 722b and 722c may be off-set or staggered (i.e., out of vertical registration) relative to one another depending upon a particular purpose. Moreover, the electrodes, e.g., 712a-712c may be staggered across or along jaw member 710 as explained in more detail below.

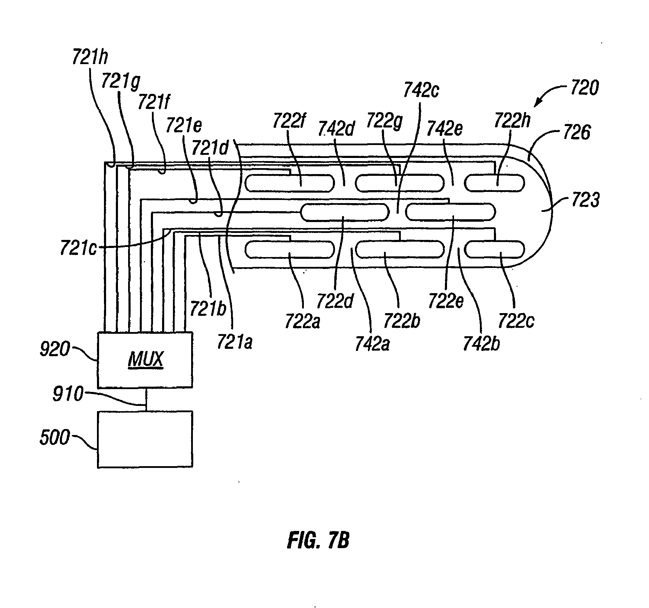

Moreover and as best shown in FIGS. 7A and 7B, a series of channels, e.g., 732a-732e or 742a-742e may be defined between the various patterns of electrodes, e.g., 712a-712h and 722a-722h, respectively, disposed on each jaw member 710 and 720. It is envisioned that the channels 732a-732e or 742a-742e are designed to control fluid flow during activation which is envisioned will create a better seal during activation. Many envisioned embodiments are described in concurrently-filed and commonly-owned U.S. Patent Application Serial No. (attorney docket no.: 2886 PCT CIP II (203-3427 PCT CIP II)) entitled "ELECTRODE ASSEMBLY FOR TISSUE FUSION," the entire contents of which being incorporated by reference herein.

Jaw members 710 and 720 operate in a similar fashion as described above with respect to FIGS. 1-5B. Jaw housings 716 and 726 may be made from an electrically and thermally insulating material such as a temperature resistant plastic or a ceramic. Alternatively, a ceramic or a so-called "cool polymer" (a thermally conductive, electrically insulative material) may be employed to regulate heat across the jaw members 710, 720 during sealing.

A series of individual leads 711a, 711b and 711c is connected to respective electrodes 712a, 712b and 712c on jaw member 710. Another series of leads 721a, 721b and 721c is connected to respective electrodes 722a, 722b and 722c on jaw member 720. The proximal ends of leads 711a-711c and 721a-721c are connected to a multiplexer (MUX) 920 which is, in turn, connected to electrosurgical generator 500 via lead 910. MUX 920 controls the electrosurgical energy to each electrode, e.g., 712a, which allows the generator 500 to automatically control the activation of individual electrodes 712a with respect to a particular sequence, a particular current density and/or a particular time. The MUX may also allow the user to selectively control the electrodes, e.g., 712a, depending upon a particular purpose or to achieve a desired surgical result.

It is also envisioned that the MUX may be configured to regulate electrode pairs, e.g., 712a and 722a, in a particular sequence, with a particular current density or for pre-set periods of time as prescribed by the generator 500 algorithm or selectively by the user. For example, during sealing it may be preferable to initially activate the distal-most pairs of electrodes 712c and 722c followed by the other electrode pairs, e.g., 712b and 722b, 712c and 722c, to progressively seal the tissue if the jaw members 710 and 720 close in a so-called "tip-biased" manner. If the jaw members 710 and 720 are configured to close in a so-called "heel-biased" manner or other particular manner, the MUX may be configured or regulated by the generator algorithm to control electrodes 712a-712c and 722a-722c differently. The MUX may also activate one electrode or a particular electrode pair at different or unequal current densities or graduated current densities depending upon a particular purpose.

FIGS. 7A and 7B show another embodiment wherein jaw member 710 includes a series of electrodes 712a-712h disposed in a longitudinal, strip like fashion on the inner facing surface of jaw member 710. For example, one pattern generally simulates a traditional staple pattern. Jaw member 720 also includes a similar pattern of electrodes (not shown) disposed on the inner facing surface thereof. Many electrode patterns are contemplated which are known to contribute to a consistent and effective end tissue seal. Some of these envisioned patterns are discussed in concurrently-filed and commonly-owned U.S. Patent Application Serial No. [203-4544] entitled "Electrode Assembly for Tissue Fusion", the entire contents of which being incorporated by reference herein.

As discussed above, each electrode, e.g., 712a, is designed to individually connect to the MUX 920 which, in turn, regulates the flow of electrosurgical energy from the generator 500 to the electrodes, e.g., 712a. The electrodes, e.g., 712a and 712f, may also be configured in pairs which together connect to the MUX 920 to regulate the sealing process depending upon a particular purpose. Moreover and as discussed above, the electrodes 712a-712f or electrode pairs may be activated in any envisioned fashion (i.e., in terms of pairings, sequence, current density, amount or time) to achieve a particular desired result and optimize sealing.

As can be appreciated, during activation, high frequency sequential switching between different pairs of electrodes regulates the sealing process to allow consistent and reliable seals to form for varying tissue types and thicknesses. It is envisioned that the MUX 920 may regulate the generator 500 to create seals in a progressive manner across or along the opposing jaw surfaces. The individual pairs of electrodes may be automatically or selectively activated sequentially, simultaneously or in any other manner to suit a particular surgical purpose. Although the time of the overall seal may increase due to various electrode pair switching algorithms, it is contemplated that more consistent current densities may be maintained across and along the entire sealing surface during the sealing process. It is envisioned that the frequency of switching between different pairs of electrodes may be increased until current fluctuations in the lead wires between the generator 500 and the multiplexer 920 become substantially equivalent to current fluctuations characteristic of a single pair of electrodes disposed on opposing jaw members 710 and 720, respectively.

It is envisioned that the bipolar forceps of the present disclosure reduces mechanical tolerance requirements of a bipolar electrosurgical forceps while maintaining or increasing current density by providing jaw members which are longer than those of the prior art. For example and as a result of the present disclosure, high frequency sequential switching between different pairs of electrodes and electrode surfaces may result in time-division multiplexing of the electrode activation process which, while lengthening the sealing time, enables design of a forceps 10 with a jaw member having a length longer than 60mm (so far as is known, 60 mm represents current mechanical limits to electrode lengths). For example, one of the issues with manufacturing jaw members 710 and 720 with electrode lengths of 60mm or greater is that the required tolerances relating to so-called "flatness" and "parallelism" must be tightly controlled along and across the electrodes. As can be appreciated, very restrictive electrode surface flatness and parallelism tolerances increase production costs. As can be appreciated, flatness and parallelism tolerances are less severe when utilizing the electrode configurations of the present disclosure.

In addition, the bipolar forceps of the present disclosure provides jaw members having a plurality of electrodes on each jaw member to form an array of individual pairs of corresponding or counterpart electrodes so that the activation sequence and electrosurgical energy applied to each electrode or each individual pair of corresponding electrodes (whether adjacent or opposing) may be varied to maintain or increase pressure of the tissue during tissue sealing, thereby increasing tissue seal integrity.

It is also contemplated that the various aforedescribed electrode arrangements may be configured for use with either an open forceps as shown in FIG. 1B or an endoscopic forceps as shown in FIG. 1A One skilled in the art would recognize that different but known electrical and mechanical considerations would be necessary and apparent to convert an open instrument to an endoscopic instrument to accomplish the same purposes as described herein.

FIG. 9 discloses a resulting tissue seal 614 sealed by electrosurgical forceps 10 or 200 of the present disclosure showing how the formation of a potentially weaker seal area due to fluid entry into the seal perimeter has been prevented or the probability of formation has been minimized. More particularly, the seal 614 illustrated in FIG. 9 is formed when the forceps 10 or 200 includes the end effector assembly 700 of FIGS. 7A through 8. Tissue 600 of lumen 602 of a patient's body such as the large or small intestines or any other passage or vessel may be performed by a heating method. The heating method may include, but is not limited to, radiofrequency (RF), ultrasonic, capacitive or thermoelectric heating methods. As previously described with respect to FIG. 6, the lumen 602 has an approximate centerline axis X-X'. The seal 614 formed by the end effector assembly 700 of staggered groups of electrodes results in a plurality of potential flow paths 616 in the areas between the electrodes which are either parallel or orthogonal to the centerline axis X-X'. The potential flow paths 616 resulting from the electrode arrangement force fluid flow to occur substantially around and substantially through flow restricting channels 732a to 732e and 742a to 742e between the individual electrodes in the electrode arrays 712a through 712h and 722a through 722h. Therefore, the seal 614 has greater reliability as compared to the seal 604 formed by an electrosurgical forceps of the prior art.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of preferred embodiments. Those skilled in the art will envision other modifications within the scope and spirit of the claims appended hereto.

## Claims

1. A bipolar electrosurgical forceps, comprising:
first and second opposing jaw members having respective inwardly facing surfaces associated therewith, the first and second jaw members adapted for relative movement between an open position to receive tissue and a closed position engaging tissue between the inwardly facing surfaces;
the first and second jaw members each including a plurality of electrodes on the inwardly facing surfaces thereof, the plurality of electrodes of the first jaw member being disposed in substantially vertical registration with the plurality of electrodes of the second jaw member;
each of the plurality of electrodes being configured to connect to a source of electrosurgical energy.

2. A bipolar electrosurgical forceps according to claim 1, wherein electrodes on at least one jaw member are grouped in pairs and each respective pair aligns with at least one electrode on the opposite jaw member.

3. A bipolar electrosurgical forceps according to claim 1, wherein the electrodes on each jaw member are grouped in pairs, each pair of electrodes on each jaw member being disposed in substantially vertical registration with a corresponding pair of electrodes on the opposite jaw member.

4. A bipolar electrosurgical forceps according to claim 1, wherein the plurality of electrodes are configured in a staggered arrangement with respect to one another on each jaw member.

5. A bipolar electrosurgical forceps according to any one of the preceding claims, wherein a series of leads couple each electrode to an electrosurgical generator via at least one multiplexer coupled therebetween.

6. A bipolar electrosurgical forceps according to claim 5, wherein the series of leads are coupled to the multiplexer and the multiplexer controls electrosurgical energy to each electrode.

7. A bipolar electrosurgical forceps according to claim 6, wherein the multiplexer controls at least one of current density and activation sequence of the electrosurgical energy to each electrode.
